# EUROPEAN PATENT SPECIFICATION

(11) **EP 3 100 053 B1**
(45) Date of publication and mention of the grant of the patent: **08.09.2021**
(21) Application number: 15743465.5
(22) Date of filing: 29.01.2015
(51) Int. Cl.: G01N 33/574, G01N 33/569

(54) **METHODS FOR THE DETECTION AND QUANTIFICATION OF CIRCULATING TUMOR CELL MIMICS**
VERFAHREN ZUR DETEKTION UND QUANTIFIZIERUNG VON MIMETIKA ZIRKULIERENDER TUMORZELLEN
PROCÉDÉS POUR LA DÉTECTION ET LA QUANTIFICATION DE MIMÉTIQUES DE CELLULES TUMORALES CIRCULANTES

(30) Priority: 31.01.2014 US 201461934600 P
(43) Date of publication of application: 07.12.2016
(73) Proprietor: Epic Sciences, Inc., San Diego, CA 92121 (US)
(72) Inventor: DITTAMORE, Ryan, San Diego, CA 92121 (US)
(74) Representative: Jones Day
(86) International application number: PCT/US2015/013452
(87) International publication number: WO 2015/116780

(56) References cited:
- WO-A1-2013/181532
- US-A1- 2009 317 836
- US-A1- 2010 297 634
- US-A1- 2010 297 634
- US-A1- 2012 276 555
- US-A1- 2013 252 259
- US-B2- 8 088 715
- JORGE NIEVA ET AL: "High-definition imaging of circulating tumor cells and associated cellular events in non-small cell lung cancer patients: a longitudinal analysis;Circulating tumor cells in non-small cell lung cancer patients", PHYSICAL BIOLOGY, INSTITUTE OF PHYSICS PUBLISHING, BRISTOL, GB, vol. 9, no. 1, 3 February 2012 (2012-02-03), page 16004, XP020218197, ISSN: 1478-3975, DOI: 10.1088/1478-3975/9/1/016004
- BALIC ET AL.: 'Circulating Tumor Cells: From Bench to Bedside' ANNU REV MED. vol. 64, 18 October 2012, pages 31 - 44, XP055215310
- Patrick Starlinger ET AL: "Discrimination between Circulating Endothelial Cells and Blood Cell Populations with Overlapping Phenotype Reveals Distinct Regulation and Predictive Potential in Cancer Therapy", NEOPLASIA, vol. 13, no. 10, 1 October 2011 (2011-10-01), pages 980-990, XP055474410, US ISSN: 1476-5586, DOI: 10.1593/neo.11916

## Description

The present disclosure relates generally to methods for the diagnosis of cancer and, more specifically, to methods for the identification and quantification of endothelial cells mimicking circulating tumor cells (CTC mimics).

### BACKGROUND

Circulating tumor cells (CTCs) and circulating endothelial cells (CECs) have recently emerged as highly promising biomarker candidates in a growing number of disease conditions, including numerous types of cancers. However, the development of full biomarker utility of CTCs and CECs has been hindered by the extremely low abundance of these cells and by the substantial heterogeneity of CTC and CEC cell populations. Due to these technical challenges the accurate identification, classification and quantification of CTCs and CECs remain very difficult today.

Currently used methods for CTC or CEC identification and quantification include flow cytometry *(e.g.,* FACS) and immunocapture technologies *(e.g.,* CellSearch®). Flow cytometry enables cell sorting but cannot robustly enumerate very small populations of cells, such as CTCs or CECs, in the presence of much more abundant cell populations, such as the white blood cell (WBC) population. Moreover, FACS-based methods do not allow for the in-depth analysis of cell morphologies.

Certain immunomagnetic capture platforms have been developed to quantify CTCs and CECs in blood samples. One example is the CellSearch® platform, which has obtained FDA-approval for the monitoring of metastatic cancer patients. The CellSearch® CTC immunocapture assay has recently been adapted for CEC detection (*see*, *e.g*., Damani, et al., 2012, Sci. Tansl. Med. 4, 126 ra33). However, CellSearch® and related platform technologies rely on an initial immunomagnetic bead-based capture step that targets a single biomarker to enrich rare cells in a sample prior to their identification and quantification. As a result, an unbiased multi-parametric analysis and classification of heterogeneous CTC or CEC cell populations that goes much beyond the targeted biomarker is not possible in enriched CTC or CEC samples.

Reported CTC and CEC levels in human blood vary greatly across the literature despite substantial assay optimization and standardization efforts. This variability in CTC and CEC assay results significantly impedes the further development of CTCs or CECs as clinically useful biomarkers. The reported variability in cell counts is generally thought to be due to highly divergent cell isolation methods and the variable immunophenotypical definition of CTCs and CECs. Moreover, CTC and CEC immunocapture methods are commonly plagued by a lack of assay sensitivity and specificity.

Nieva J. et al., Physical Biol. 9:16004 (2012), for instance, relates to a method using enrichment free fluorescent labeling of CTCs followed by automated digital microscopy in patients with non-small cell lung cancer and classification as a CTC or not based on cell morphology and immunophenotype of cells stained with anti-cytokeratin antibodies and anti-CD45 antibody.

Thus, there exists a need for improved methods for CTC and CEC detection, classification and quantification. The present disclosure addresses this need by providing methods for detecting CTC-mimicking CEC subpopulations (CTC mimics) in non-enriched blood samples of cancer patients. Related advantages are provided as well.

### SUMMARY

Generally, the present disclosure describes methods of distinguishing circulating tumor cells (CTCs) from CTC mimics.

In one aspect, disclosed herein is a method of distinguishing circulating tumor cells (CTCs) from CTC mimics, comprising: (a) determining the presence or absence of one or more immunofluorescent CTC markers in nucleated cells in a non-enriched blood sample to detect a CTC candidate, (b) determining the presence or absence of one or more immunofluorescent CEC markers in the CTC candidate, and (c) assessing the morphology of the CTC candidate, wherein CTCs are distinguished from CTC mimics based on a combination of distinct immunofluorescent staining and morphological characteristics.

In some cases, (a) further comprises determining the presence or absence of one or more immunofluorescent sample cell markers in the nucleated cells.

In some cases, the distinct immunofluorescent staining of CTCs includes the presence of an immunofluorescent CTC marker, the absence of an immunofluorescent CEC marker, and the absence of an immunofluorescent sample cell marker.

In some cases, the distinct immunofluorescent staining of CTC mimics includes the presence of an immunofluorescent CTC marker, the presence of an immunofluorescent CEC marker, and the absence of an immunofluorescent sample cell marker.

Specifically, the present invention provides to a method of distinguishing circulating tumor cells (CTCs) from CTC mimics based on a combination of distinct immunofluorescent staining and morphological characteristics, wherein the method comprises (a) determining the presence or absence of one or more immunofluorescent CTC markers in nucleated cells in a non-enriched blood sample to detect a CTC candidate, wherein the non-enriched blood sample is a sample not enriched for any specific population or subpopulation of nucleated cells; (b) determining the presence or absence of one or more immunofluorescent circulating endothelial cell (CEC) markers in the CTC candidate; (c) determining the presence or absence of one or more immunofluorescent sample cell markers in the CTC candidate; and (d) assessing the morphology of the CTC candidate, wherein the immunofluorescent CTC markers comprise a cytokeratin (CK), wherein the immunofluorescent CEC markers comprise Von Willebrand factor (vWF), wherein the immunofluorescent sample cell markers comprise cluster of differentiation 45 (CD45), wherein the distinct immunofluorescent staining of CTC mimics includes the presence of the immunofluorescent CTC marker CK (CK+), the presence of the immunofluorescent CEC marker vWF (vWF+), and the absence of the immunofluorescent sample cell marker CD45 (CD45-), and wherein the distinct immunofluorescent staining of CTCs includes the presence of CK (CK+), the absence of vWF (vWF-), and the absence of CD45 (CD45-).

In one embodiment of the method of the invention, the CK includes cytokeratin 1, 4, 5, 6, 7, 8, 10, 13, 18 or 19, or a combination thereof.

In another embodiment of the method of the invention, determining the presence of CK in the CTC candidate comprises identifying nucleated cells having a relative CK expression of >3, wherein the relative expression level is expressed by comparing the fluorescence signal of a cell that is positive for CK with the corresponding fluorescence signal of surrounding cells that are negative for CK.

In another embodiment of the method of the present invention, the immunofluorescent CEC markers include CD31, CD34, CD105, or CD146.

In another embodiment of the method of the present invention, determining the presence of vWF in the CTC candidate comprises identifying CTC candidates having a relative vWF expression of >6, wherein the relative expression level is expressed by comparing the fluorescence signal of a cell that is positive for vWF with the corresponding fluorescence signal of surrounding cells that are negative for vWF.

In another embodiment of the method of the present invention, assessing the morphology of the CTC candidate comprises assessing the CTC candidate by nuclear detail, nuclear contour, presence or absence of nucleoli, quality of cytoplasm, quantity of cytoplasm, or immunofluorescent staining patterns.

In another embodiment of the method of the present invention, the blood sample was obtained from a non-small cell lung cancer (NSCLC) patient.

In another embodiment of the method of the present invention, the method is performed by fluorescent scanning microscopy. Specifically, the microscopy provides a field of view comprising more than 2, 5, 10, 20, 30, or 50 CTC candidates, wherein the CTC candidate is surrounded by more than 10, 50, 100, 150 or 200 white blood cells.

Hereinafter, reference to a method of the present invention shall mean a method of distinguishing CTCs from CTC mimics as defined above.

In some cases, the immunofluorescent sample cell markers are specific for white blood cells (WBCs). In some embodiments, the immunofluorescent sample cell markers comprise CD 45.

In some cases, the method further comprises the initial step of obtaining a blood sample from a patient. In some embodiments, the blood sample was obtained from a non-small cell lung cancer (NSCLC) patient.

In some embodiments, the method is performed by fluorescent scanning microscopy. In some embodiments, the microscopy provides a field of view comprising more than 2, 5, 10, 20, 30, 40 or 50 CTC candidates, wherein each CTC candidate is surrounded by more than 10, 50, 100, 150 or 200 WBCs.

In some embodiments, determining the presence or absence of the immunofluorescent CTC markers comprises comparing the distinct immunofluorescent staining of CTC candidates with the distinct immunofluorescent staining of WBCs.

In some embodiments, determining the presence or absence of the immunofluorescent CEC markers comprises comparing the distinct immunofluorescent staining of CTC candidates with the distinct immunofluorescent staining of WBCs.

In some cases, the immunofluorescent CTC markers comprise a cytokeratin (CK). In some cases, determining the presence of CK in nucleated cells comprises identifying nucleated cells having a relative CK expression of >3.

In some cases, the immunofluorescent CEC markers comprise Von Willebrand factor (vWF), cluster of differentiation (CD) 31, CD 34, CD 105, CD 145 or CD 146. In some cases, determining the presence of vWF in CTC candidates comprises identifying CTC candidates having a relative vWF expression of >6.

In some embodiments, assessing the morphology of the CTC candidate comprises comparing the morphological characteristics of the CTC candidate with the morphological characteristics of surrounding WBCs.

In some embodiments, assessing the morphology of the CTC candidate comprises comparing the morphological characteristics of the CTC candidate with the morphological characteristics of a CTC.

In some embodiments, assessing the morphology of the CTC candidate comprises comparing the morphological characteristics of the CTC candidate with the morphological characteristics of a CEC.

In some embodiments, the morphological characteristics comprise nucleus size, nucleus shape, cell size, cell shape, nuclear to cytoplasmic ratio. In some embodiments, assessing the morphology of the CTC candidate comprises assessing the CTC candidate by nuclear detail, nuclear contour, presence or absence of nucleoli, quality of cytoplasm, quantity of cytoplasm, or immunofluorescent staining patterns.

In some embodiments, the method further comprises quantifying the number of CTC candidates in the blood sample.

In some embodiments, the method further comprises quantifying the number of CTC mimics in the blood sample.

In some embodiments, the method further comprises quantifying the number of CTCs in the blood sample, comprising i) quantifying the number of CTC candidates in the blood sample, ii) quantifying the number of CTC mimics in the blood sample, and iii) subtracting the number of CTC mimics from the number of CTC candidates to quantify the number of CTCs in the blood sample.

In another aspect, described herein is a method of improving the accuracy and specificity of CTC quantifications in a blood sample, comprising i) quantifying the number of CTC candidates in the blood sample to obtain an approximate CTC count, ii) distinguishing circulating tumor cells (CTCs) from CTC mimics according to a method of this disclosure, iii) quantifying the number of CTC mimics in the blood sample, and iv) subtracting the number of CTC mimics from the number of CTC candidates to improve the accuracy and specificity of CTC quantifications in the blood sample.

In another aspect, described herein is a method of monitoring an anti-cancer treatment response in a patient, comprising i) collecting two or more blood samples from the patient at different time-points throughout a treatment period; and ii) quantifying the number of CTC mimics in each blood sample according to a method of this disclosure, wherein an increasing number of CTC mimics in the blood samples collected at different time-points throughout the treatment period indicates a positive treatment response in the patient.

In another aspect, described herein is a method of determining the efficacy of an anti-cancer treatment in a patient, comprising i) collecting blood samples from a population of patients receiving the anti-cancer treatment, ii) collecting blood samples form a population of patients not receiving the anti-cancer treatment, and iii) quantifying the number of CTC mimics in each blood sample according to a method of this disclosure, wherein elevated numbers of CTC mimics in the blood samples from the population of patients receiving the anti-cancer treatment relative to the blood samples from the populations of patients not receiving the anti-cancer treatment indicates that the anti-cancer treatment is efficacious.

### BRIEF DESCRIPTION OF THE DRAWINGS

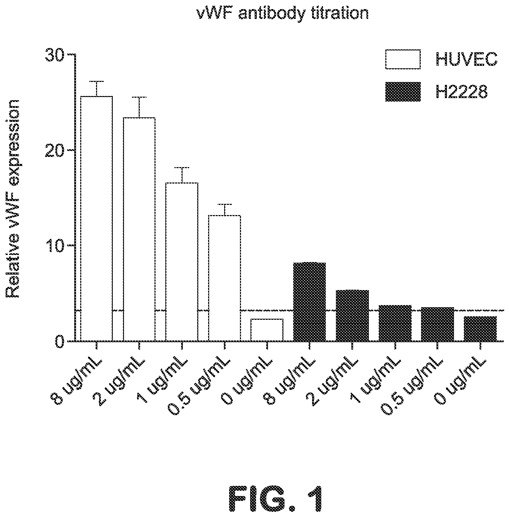

**FIG. 1** shows the results of an exemplary Von Willebrand Factor (vWF) antibody titration experiment. A primary vWF antibody was tested in a serial dilution using human umbilical vein endothelial cells (HUVECs, grey bars, left) and H2228 adenocarcinoma cells (black bars, right). Data are plotted as means +/- SEM.

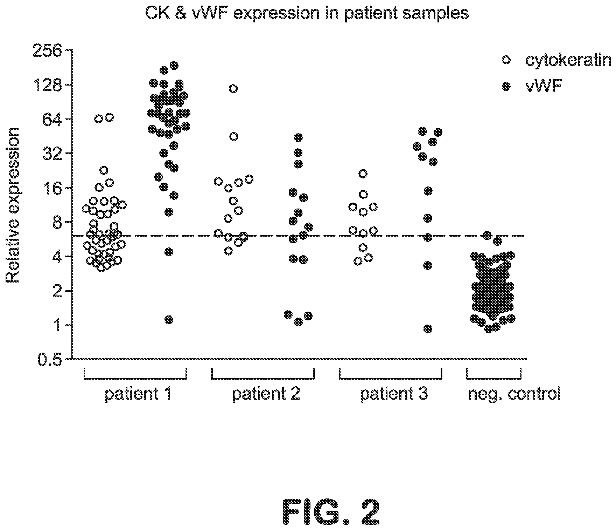

**FIG. 2** shows the results of an exemplary experiment to determine cytokeratin (CK) and Von Willebrand Factor (vWF) expression in blood samples of three non-small cell lung carcinoma (NSCLC) patients. The blood samples were analyzed by fluorescent scanning microscopy using CK- and vWF-specific antibodies. CK and vWF expressing cell populations are shown for each patient. CK expressing cells are shown as grey dots *(i.e.,* left column for each patient); vWF expressing cells are shown as black dots *(i.e.,* right column for each patient). For the negative control experiment, a H441 lung carcinoma cell line was used that does not express vWF and therefore does not show specific vWF staining. All CTC candidates showing a relative CK expression >3 are plotted. The dotted line marks relative CK or vWF expression levels >6.

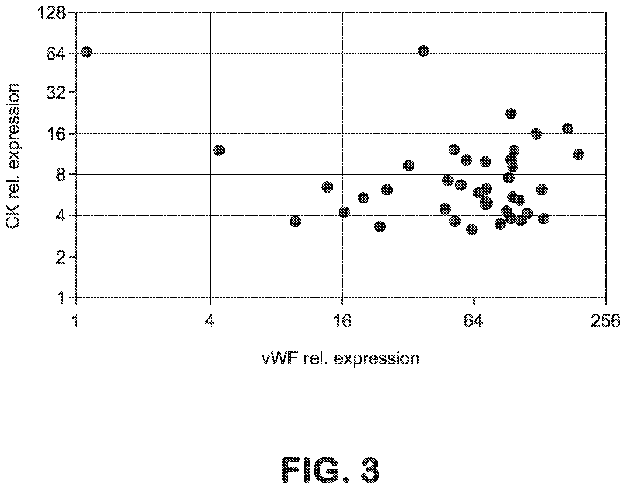

**FIG. 3** shows a scatter plot illustrating the relative cytokeratin (CK) and Von Willebrand Factor (vWF) expression in CK-expressing cells that were observed in an exemplary blood sample from a non-small cell lung carcinoma (NSCLC) patient. A subpopulation of CK-expressing cells co-expresses vWF.

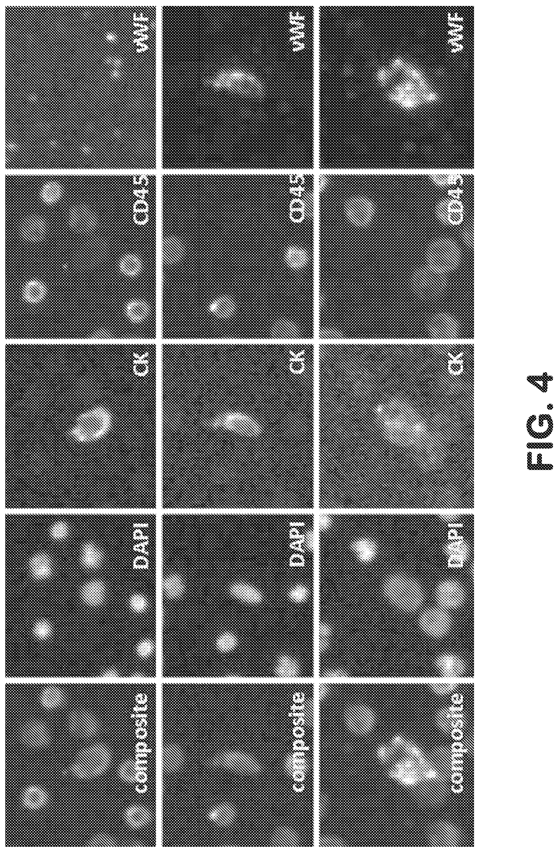

**FIG. 4** shows representative images of morphologically distinct CTCs (CK⁺/vWF⁻ cells; top row), CTC mimics (CK⁺/vWF⁺ cells; center row) and CECs (CK⁻/vWF⁺ cells; bottom row) that were observed in the blood sample of a non-small cell lung carcinoma (NSCLC) patient. Columns from right to left show vWF-staining, CD45-staining, CK-staining, DAPI-staining and composite images.

### DETAILED DESCRIPTION

The present disclosure is based, in part, on the unexpected discovery of CECs in the blood samples of cancer patients that mimic CTCs with respect to certain aspects of their immunofluorescent staining and their morphological characteristics *(i.e.,* CTC mimics).

The present disclosure is further based, in part, on the surprising discovery that CTC mimics have value in their own right as diagnostic and prognostic indicators of treatment responses in cancer patients. Without wishing to be bound by theory, it is believed that increasing levels of CTC mimics in a cancer patient undergoing anti-cancer treatment reflect the breaking up of vasculature of a patient's tumor in response to the treatment and is predictive of a positive treatment response.

The present disclosure is further based, in part, on the discovery that CTC mimics can be detected in non-enriched blood samples by combining the detection of one or more immunofluorescent CTC markers and one or more immunofluorescent CEC markers in the nucleated cells of a non-enriched blood sample with an assessment of the morphology of the nucleated cells. The present disclosure is further based, in part, on the discovery that CECs can be detected in non-enriched blood samples by comparing the immunofluorescent marker staining and morphological characteristics of CTC mimics with the immunofluorescent marker staining and morphological characteristics of CTCs, CECs, or WBCs.

A fundamental aspect of the present disclosure is the robustness of the disclosed methods. The rare event detection (RED) disclosed herein with regard to CTC mimics, CECs, and CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis but that instead compare enriched populations with inherently distorted contextual comparisons of rare events.

The disclosure generally relates to methods for distinguishing CTCs from CTC mimics (*i.e.,* CECs mimicking CTCs) in populations of CTC candidates in non-enriched blood samples and for monitoring anti-cancer treatment responses in cancer patients. One major advantage of the present disclosure is the improvement in the accuracy and sensitivity of CTC detection and quantification. CTC mimics frequently present as false positives in CTC assays and lower CTC assay specificity and accuracy. Thus, by enabling the detection of CTC mimics and the differentiation of CTC mimics from CTCs, the methods of this disclosure help to eliminate false positive CTC counts in CTC assays and thereby improve CTC assay performance. Accurate and sensitive CTC assays are needed to realize the full potential of CTCs as diagnostic and prognostic biomarkers in cancer.

Another major advantage of the present disclosure is that the methods provided are useful for the identification, quantification and further characterization of CEC mimics as biomarkers in their own right that are useful, *e.g.,* for monitoring treatment responses in cancer patients.

The present disclosure is of particular benefit to cancer patients because the methods provided improve diagnostic approaches relying on CTC quantification and enable the detection and further characterization of CTC mimics, which are promising biomarkers in their own right. Specifically, cancer patients will benefit from the improved diagnosis of their disease and from the improved tailoring of their treatment regimens, which will result from the application of high-performance biomarker assays for CTCs and CTC mimics.

Disclosed herein are methods of distinguishing circulating tumor cells (CTCs) from CTC mimics, including (a) determining the presence or absence of one or more CTC biomarkers in nucleated cells in a non-enriched blood sample to detect a CTC candidate, (b) determining the presence or absence of one or more CEC biomarkers in the CTC candidate, and (c) assessing the morphology of the CTC candidate, whereby the CTCs are distinguished from CTC mimics based on a combination of distinct biomarker staining and morphological characteristics.

Disclosed herein are methods for distinguishing circulating tumor cells (CTCs) from CTC mimics, including (a) determining the presence or absence of one or more immunofluorescent CTC markers in nucleated cells in a non-enriched blood sample to detect a CTC candidate, (b) determining the presence or absence of one or more immunofluorescent CEC markers in the CTC candidate, and (c) assessing the morphology of the CTC candidate, wherein CTCs are distinguished from CTC mimics based on a combination of distinct immunofluorescent staining and morphological characteristics. In some examples, (a) further includes determining the presence or absence of one or more immunofluorescent sample cell markers in the nucleated cells.

It must be noted that, as used in this specification and the appended claims, the term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

The biological samples of this disclosure can be any sample suspected to contain CTCs or CTC mimics, including solid tissue samples, such as bone marrow, and liquid samples, such as whole blood, plasma, amniotic fluid, pleural fluid, peritoneal fluid, central spinal fluid, urine, saliva and bronchial washes. According to the method of the present invention, the biological sample is a blood sample. As will be appreciated by those skilled in the art, a biological sample can include any fraction or component of blood, without limitation, T-cells, monocytes, neutrophils, erythrocytes, platelets and microvesicles such as exosomes and exosome-like vesicles.

The biological samples of this disclosure can be obtained from any organism, including mammals such as humans, primates (*e.g.,* monkeys, chimpanzees, orangutans, and gorillas), cats, dogs, rabbits, farm animals (*e.g.,* cows, horses, goats, sheep, pigs), and rodents (*e.g.,* mice, rats, hamsters, and guinea pigs).

It is noted that, as used herein, the terms "organism," "individual," "subject," or "patient" are used as synonyms and interchangeably.

The organisms of this disclosure include, for example, any organism having cancer, suspected of having cancer or suspected of being at risk of developing cancer. In some embodiments, the organism is a human cancer patient. In some embodiments, the organism is receiving an anti-cancer treatment. In some embodiments, the organism has discontinued an anti-cancer treatment. In some embodiments, the organism is treatment naive.

Anti-cancer treatments include, for example and without limitation, surgery, drug therapy (*e.g.,* chemotherapy), radiation therapy, or combinations thereof.

The organism can be a healthy organism, including for example and without limitation, a healthy individual or a non-cancer patient in the control group of a clinical study, a cured cancer patient or an individual being at risk of developing cancer. Elevated risks for developing cancer can, *e.g.,* be due to a genetic predisposition for cancer (*e.g.,* BRCA 1 or BRCA 2 mutations), a family history of cancer or exposure to carcinogens (*e.g.,* a cigarette smoke, exhaust fumes, smog, asbestos, environmental pollution or toxins).

In some embodiments, the organism is an animal model for cancer, including, without limitation, a xenograft mouse model, a transgenic mouse carrying a transgenic oncogene, a knockout mouse lacking a proapoptotic gene and others. A person of ordinary skill understands that many other animal models for cancer conditions (in mice or other organisms) are well known in the art.

The tumors or cancers of this disclosure are typically solid tumors or cancers. The tumors can be primary tumors or metastatic tumors. The tumors can be vascularized.

The cancers of this disclosure include, without limitation, lung cancer (*e.g*., small-cell lung cancer (SCLC), non-small cell lung cancer (NSCLC, including, *e.g*., adenocarcinomas or lung carcinoid tumor), skin cancer, colon cancer, renal cancer, liver cancer, pancreatic cancer, thyroid cancer, bladder cancer, gall bladder cancer, brain cancer *(e.g.,* glioma, glioblastoma, medulloblastoma, neuroblastoma), breast cancer, ovarian cancer, endometrial cancer, prostate cancer, testicular cancer and lymphomas (*e.g.,* Hodgkin's lymphoma, non-Hodgkin's lymphoma, T-cell lymphoma, B-cell lymphoma). The cancers can include cancers of all stages, e.g., stage 1, stage 11, stage III, or stage IV cancers. The cancers can be at least partly responsive to therapy (*e.g.,* surgery, chemotherapy, radiation therapy) or unresponsive. The cancers can be resistant to one or more anti-cancer treatments (*e.g.,* specific chemotherapy regimens).

In some embodiments, the blood sample was obtained from a cancer patient. In some embodiments, the cancer patient received an anti-cancer treatment for a period of time *(e.g.,* for more than 1 day, 1 week, 1 month, 3 months, 6 months, 9 months, 1 year, 2 years, 3 years, 4 years, 5 years). In some embodiments the blood sample is a plurality of blood samples. In some embodiments the plurality of blood samples were collected over a period of time. In some embodiments, at least one blood sample from the plurality of blood samples was collected before the cancer patient received an anti-cancer treatment for a period of time. In some embodiments, at least one blood sample from the plurality of blood samples was obtained when the cancer patient was treatment naive. In some embodiments, at least one blood sample of the plurality of blood samples was obtained from a cancer patient during the period of time when the cancer patient received an anti-cancer treatment. In some embodiments, at least one blood sample of the plurality of blood samples was obtained before the cancer patient received an anti-cancer treatment for a period of time and at least one blood sample of the plurality of blood samples was obtained during the period of time when the cancer patient received the anti-cancer treatment. In some embodiments, a first blood sample was obtained at a first time during the period of time when the cancer patient received an anti-cancer treatment and a second blood sample was obtained at a second time during the period of time when the cancer patient received the anti-cancer treatment. In some embodiments the first time and the second time were separated by a period of time of more than 1 day, 1 week, 2 weeks, 1 month, 2 months, 3 months, 6 months, 9 months, 1 year, 2 years, 3 years, 4 years, or 5 years.

In some embodiments, the blood sample was obtained from a non-small cell lung cancer (NSCLC) patient.

In some aspects, the methods further include the initial step of obtaining a blood sample from a patient.

The samples of this disclosure may each contain a plurality of cell populations and cell subpopulation that are distinguishable by methods well known in the art (*e.g.,* FACS, immunohistochemistry). For example, a blood sample may contain populations of non-nucleated cells, such as erythrocytes (*e.g.,* 4-5 million/µl) or platelets (150,000-400,000 cells/µl), and populations of nucleated cells such as white blood cells (WBCs, *e.g.,* 4,500-10,000 cells/µl), CECs or CTCs (circulating tumor cells; *e.g.,* 2-800 cells/µl). WBCs may contain cellular subpopulations of, *e.g*., neutrophils (2,500-8,000 cells/µl), lymphocytes (1,000-4,000 cells/µl), monocytes (100-700 cells/µl), eosinophils (50-500 cells/µl), basophils (25-100 cells/ µl) and the like. The samples of this disclosure are non-enriched samples, *i.e.,* they are not enriched for any specific population or subpopulation of nucleated cells. For example, non-enriched blood samples are not enriched for CTCs, CTC candidates, CTC mimics, CECs, WBCs, B-cells, T-cells, NK-cells, monocytes, or the like.

The term "rare cell," as used herein, refers to a cell that has an abundance of less than 1:1,000 in a cell population, *e.g*., an abundance of less than 1:5,000, 1:10,000, 1:30,000, 1:50:000, 1:100,000, 1:300,000, 1:500,000, or 1:1,000,000. In some cases, the rare cell has an abundance of 1:50:000 to 1:100,000 in the cell population. In some embodiments, the rare cell is a CTC, CTC candidate, CTC mimic or CEC.

The samples of this disclosure can be obtained by any applicable method known to a person of skill, including, *e.g.,* by solid tissue biopsy or fluid biopsy (*see, e.g.,* Marrinucci D. et al., 2012, Phys. Biol. 9 016003; Nieva J.. et al., 2012, Phys. Biol. 9 016004). A blood sample can be extracted from any source known to include blood cells or components thereof, such as venous, arterial, peripheral, tissue, cord and the like. The sample can be processed using well known and routine clinical methods (*e.g.,* procedures for drawing and processing whole blood). In some cases, a blood sample is drawn into anti-coagulant blood collection tubes (BCT), which can contain EDTA or Streck Cell-Free DNA™. In other cases, a blood sample is drawn into CellSave® tubes (Veridex). A blood sample can be stored for up to 12 hours, 24 hours, 36 hours, 48 hours, 60 hours, or 96 hours before further processing.

In some embodiments, the methods of this disclosure comprise obtaining a white blood cell (WBC) count for the blood sample. In certain embodiments, the WBC count may be obtained by using a HemoCure® WBC device (Hemocure, Ängelholm, Sweden).

In some embodiments, the methods of this disclosure comprise a step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, *e.g*., in a PBS solution.

In some embodiments, nucleated cells from a sample, such as a blood sample, are deposited as a monolayer on a planar support. The planar support can be of any material, *e.g.,* any fluorescently clear material, any material conducive to cell attachment, any material conducive to the easy removal of cell debris, any material having a thickness of < 100 µm. In some embodiments, the material is a film. In some embodiments the material is a glass slide. The glass slide can be coated to allow maximal retention of live cells (*See*, *e.g.,* Marrinucci D. et al., 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise an initial step of depositing nucleated cells from the blood sample as a monolayer on a glass slide. In certain embodiments, the method comprises depositing about 3 million cells onto a glass slide. In some embodiments, the WBC count is used to determine the amount of blood required to plate a consistent loading volume of nucleated cells per glass slide.

In some embodiments, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI).

The Circulating Tumor Cells (CTCs) of this disclosure are tumor cells that are circulating in the bloodstream of an organism. Circulating Endothelial Cells (CECs) of this disclosure are endothelial cells that are circulating in the bloodstream of an organism.

CTC mimics are CECs that share certain biomarkers (CTC markers) or certain morphological characteristics (*e.g.,* nucleus-to-cytoplasm ratio), or combinations thereof, with CTCs. CTC mimics are likely to be classified incorrectly as CTCs in CTC assays that primarily rely on the detection of the biomarkers or morphological characteristics, or combinations thereof, that are shared between CTC mimics and CTCs. As a result, CTC mimics are likely to present as false positives in such CTC assays and lower their specificity and accuracy.

The term "CTC candidate," as used herein, refers to a cell that is detected based on a biomarker (CTC marker) or a morphological characteristic (*e.g.,* nucleus-to-cytoplasm ratio), or combination thereof, that is shared between CTC mimics and CTCs. A CTC candidate can be a CTC or a CTC mimic. In a population of nucleated cells a subpopulation of CTC candidates comprises any cell that can either be a CTC or a CTC mimic. Thus, CTC candidate subpopulations can, for example, be composed of 100% CTCs or 100% CTC mimics or any combination of CTCs and CTC mimics (*e.g.,* 1% CTCs and 99% CTC mimics, 50% CTCs and 50% CTC mimics, or 99% CTCs and 1% CTC mimics).

According to this disclosure, CTCs, CTC candidates, CTC mimics and CECs are detected among the nucleated cells of a sample based on a combination of distinct biomarkers and morphological characteristics.

The term "CTC mimic" as used herein, refers to a cell that, while sharing one or more biomarkers, morphological characteristics (*e.g.,* nucleus-to-cytoplasm ratio), or a combination thereof, with a CTC, is not a CTC.

CEC markers are biomarkers that can be used to detect CECs, but not CTCs. In some embodiments, the CEC marker is present in CECs, CTC mimics and CTC candidates and absent in CTCs.

CEC markers include, without limitation, any biomarker that is specific for endothelial cells (*e.g.,* cluster of differentiation (CD) 146, Von Willebrand factor (vWF), CD 31, CD 34, or CD 105).

CTC markers are biomarkers that can be used to detect CTCs, but not CECs. In some embodiments, the CTC marker is present in CTCs, CTC candidates and CTC mimics and absent in CECs.

CTC markers can include any cancer-specific biomarker. Cancer-specific biomarkers include biomarkers that are specific for a given cancer-type of interest (*e.g.,* non-small cell lung cancer, NSCLC), a clinical cancer-stage of interest (*e.g.,* stage IV), or a cancer cell property of interest (*e.g.,* energy metabolism, epithelial-mesenchymal transition). Additionally, cancer-specific biomarkers include more general cancer markers, such as cancer markers that are present in several cancer-types, but not in normal cells, or cancer markers that signal the malignant transformation of a cell. A person of skill will recognize that many specific and general tumor-specific biomarkers are known in the art.

CTC markers include, for example and without limitation, anaplastic lymphoma kinase (ALK), androgen receptor (AR), Axl, cMET, cytokeratins 1, 4, 5, 6, 7, 8, 10, 13, 18 or 19; CD 31, CD 99, CD 117, chromatogranin, desmin, E-cadherin, epidermal growth factor receptor (EGFR), epithelial cell adhesion molecule (EpCAM), epithelial membrane antigen (EMA), gross cystic disease fluid protein (GCDFP-15), HMB-45, inhibin, MART-1, MCM2, Myo D1, muscle-specific actin (MSA), N-cadherin, neurofilament, neuron-specific enolase (NSE), p63, placental alkaline phosphatase (PLAP), prostate specific membrane antigen (PSMA), S100 protein, smooth muscle actin (SMA), synaptophysin, thyroid transcription factor-1 (TTF-1), tumor M2-PK (*i.e.,* pyruvate kinase isoenzyme type M2), vimentin and more.

Sample cell markers are biomarkers that are present in at least one cell-type in a sample, but that are not present in CECs, CTCs, CTC candidates and CTC mimics. In some embodiments, the sample cell marker is present in at least one cell-type in the sample and absent in CECs, CTCs, CTC candidates and CTC mimics. In some embodiments, the sample cell marker is present in a cell type in the sample that is more abundant than CECs, CTCs, CTC candidates, or CTC mimics. In some embodiments, the sample cell marker is present in a WBC and absent in CECs, CTCs, CTC candidates and CTC mimics. In the method of the invention, the sample cell marker is CD 45.

The term "biomarker," as used herein, refers to a biological molecule, or a fragment of a biological molecule, the change and/or the detection of which can be correlated with a particular physical condition or state of a CTC, CTC candidate, CTC mimic, or CEC. The terms "marker" and "biomarker" are used interchangeably throughout the disclosure. Such biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e.g.,* glycoproteins, ribonucleoproteins, lipoproteins). The term also encompasses portions or fragments of a biological molecule, for example, peptide fragment of a protein or polypeptide

A person skilled in the art will appreciate that a number of methods can be used to determine the presence or absence of a biomarker, including microscopy based approaches, such as fluorescence microscopy or fluorescence scanning microscopy (*see, e.g.,* Marrinucci D. et al., 2012, Phys. Biol. 9 016003; Nieva J.. et al., 2012, Phys. Biol. 9 016004). Other approaches include mass spectrometry, gene expression analysis (*e.g.,* gene-chips, PCR, FISH) and antibody-based approaches, including immunofluorescence, immunohistochemistry, immunoassays (*e.g.,* Western blots, enzyme-linked immunosorbent assay (ELISA), immunoprecipitation, radioimmunoassay, dot blotting, and FACS). In some embodiments, the methods of this disclosure are performed in an automated or robotic fashion. In some embodiments, the signal from multiple samples are detected simultaneously.

A person of skill in the art will further appreciate that the presence or absence of biomarkers in a cell can be detected using any class of marker-specific binding reagents known in the art, including, *e.g*., antibodies, aptamers, fusion proteins, such as fusion proteins including protein receptor or protein ligand components (*e.g.* CD 146, vWF, CD 31, CD 34, CD 105, or CD 45-binding receptors or ligands), or biomarker-specific peptides and small molecule binders.

In some embodiments, the presence or absence of vWF, CD 146, CD 31, CD 34, CD 105, CD 45 or a cytokeratin (*e.g.,* cytokeratin 1, 4, 5, 6, 7, 8, 10, 13, 18 or 19), or a combination thereof, is determined by an antibody. In some embodiments, the presence or absence of vWF and one or more cytokeratins (*e.g.,* cytokeratin 1, 4, 5, 6, 7, 8, 10, 13, 18 or 19) is determined by an antibody. In some embodiments, the presence or absence of vWF, one or more cytokeratin (*e.g.,* cytokeratin 1, 4, 5, 6, 7, 8, 10, 13, 18 or 19) or CD 45 is determined by an antibody.

The antibodies of this disclosure bind specifically to a biomarker. In some cases, the antibodies bind specifically to a single biomarker (*e.g.,* cytokeratin 1). In other cases, the antibodies are pan-specific. Pan-specific antibodies of this disclosure can bind specifically to one or more members of a biomarker family (*e.g.,* one or more members of the cytokeratin family, including cytokeratins 1, 4, 5, 6, 7, 8, 10, 13, 18 and 19). The antibody can be any immunoglobulin or derivative thereof, whether natural or wholly or partially synthetically produced. All antibody derivatives which maintain specific binding ability can also be used. The antibody has a binding domain that is homologous or largely homologous to an immunoglobulin binding domain and can be derived from natural sources, or partly or wholly synthetically produced. The antibody can be a monoclonal or polyclonal antibody. In some cases, the antibody is a single-chain antibody. In some cases, the antibody includes a single-chain antibody fragment. In some cases, the antibody can be an antibody fragment including, but not limited to, Fab, Fab', F(ab')2, scFv, Fv, dsFv diabody, and Fd fragments. Due to their smaller size antibody fragments can offer advantages over intact antibodies in certain applications. Alternatively or additionally, the antibody can comprise multiple chains which are linked together, for example, by disulfide linkages, and any functional fragments obtained from such molecules, wherein such fragments retain specific-binding properties of the parent antibody molecule. Those of ordinary skill in the art will appreciate that the antibody can be provided in any of a variety of forms including, for example, humanized, partially humanized, chimeric, chimeric humanized, *etc.* The antibody can be prepared using any suitable methods known in the art. For example, the antibody can be enzymatically or chemically produced by fragmentation of an intact antibody or it can be recombinantly produced from a gene encoding the partial antibody sequence.

A wide variety of detectable labels can be used for the direct or indirect detection of biomarkers. Suitable detectable labels include, but are not limited to, fluorescent dyes (*e.g.,* fluorescein, fluorescein isothiocyanate (FITC), Oregon Green™, rhodamine, Texas Red, tetrarhodamine isothiocynate (TRITC), Cy3, Cy5, Alexa Fluor® 647, Alexa Fluor® 555, Alexa Fluor® 488), fluorescent protein markers (*e.g.,* green fluorescent protein (GFP), phycoerythrin, etc.), enzymes (*e.g.,* luciferase, horseradish peroxidase, alkaline phosphatase, etc.), nanoparticles, biotin, digoxigenin, metals, and the like.

In some embodiments, the biomarkers are immunofluorescent markers. In some embodiments, the biomarkers are immunofluorescent CTC markers. In some embodiments, the biomarkers are immunofluorescent CEC markers.

In the method of the invention, the immunofluorescent CTC markers include a cytokeratin (CK). Cytokeratins include, *e.g*., cytokeratin 1, 4, 5, 6, 7, 8, 10, 13, 18 or 19. In some embodiments, the immunofluorescent CTC marker is a plurality of cytokeratins, including two or more of cytokeratins 1, 4, 5, 6, 7, 8, 10, 13, 18 or 19.

In some cases, the immunofluorescent CEC markers include Von Willebrand factor (vWF) cluster of differentiation (CD) 31, CD 34, CD 105, CD 145 or CD 146.

In some cells the sample cell markers are immunofluorescent sample cell markers. In some embodiments, the immunofluorescent sample cell markers are specific for white blood cells (WBCs). In the method of the invention, the immunofluorescent sample cell markers comprise CD 45.

The distinct immunofluorescent staining of nucleated cells of a sample includes the presence or absence of immunofluorescent markers, such as immunofluorescent CTC markers, immunofluorescent CEC markers and immunofluorescent sample cell markers.

In some cases, the distinct immunofluorescent staining of CTCs includes the presence of an immunofluorescent CTC marker, the absence of an immunofluorescent CEC marker, and the absence of an immunofluorescent sample cell marker. According to the invention, the distinct immunofluorescent staining of CTCs includes positive staining for CK, negative staining for vWF and negative staining for CD45 (CK⁺/vWF⁻/CD45⁻). *See, e.g.,* **Example 1,** **FIG. 4** (CTCs (CK⁺/vWF⁻): top row).

In some cases, the distinct immunofluorescent staining of CECs includes the presence of an immunofluorescent CEC marker, the absence of an immunofluorescent CTC marker and the absence of an immunofluorescent sample cell marker. According to the invention, the distinct immunofluorescent staining of CECs includes positive staining for vWF, negative staining for CK and negative staining for CD45 (vWF⁺/CKVCD45⁻). *See, e.g.,* **Example 1,** **FIG. 4** (CECs (CK-/vWF+): bottom row).

In some cases, the distinct immunofluorescent staining of CTC mimics includes the presence of an immunofluorescent CTC marker, the presence of an immunofluorescent CEC marker, and the absence of an immunofluorescent sample cell marker. According to the invention, the distinct immunofluorescent staining of CTC mimics includes positive staining for CK, positive staining for vWF and negative staining for CD45 (CK⁺/vWF⁺/CD45⁻). *See, e.g.,* **Example 1,** **FIG. 4** (CTC mimics (CK⁺/vWF⁺): center row).

In some cases, the distinct immunofluorescent staining of CTC candidates includes the presence of an immunofluorescent CTC marker, the absence of an immunofluorescent CEC marker, and the absence of an immunofluorescent sample cell marker. In other cases, the distinct staining of CTC candidates includes the presence of an immunofluorescent CTC marker, the presence of an immunofluorescent CEC marker, and the absence of an immunofluorescent sample cell marker. According to the invention, the distinct immunofluorescent staining of CTC candidates includes positive staining for CK and negative staining for CD45 (CK⁺/CD45⁻).

In some cases, the distinct immunofluorescent staining of a sample cell includes the presence of an immunofluorescent sample cell marker, the absence of an immunofluorescent CEC marker and the absence of an immunofluorescent CTC marker.

In some embodiments, the distinct immunofluorescent staining of a CEC, CTC, CTC mimic, CTC candidate or sample cell includes distinct intracellular staining patterns for an immunofluorescent CEC marker, an immunofluorescent CTC marker, or an immunofluorescent sample cell marker. For example, the intracellular staining for an immunofluorescent marker of this disclosure can be distinctly diffuse, punctuate, cytoplasmic, nuclear or membrane bound.

In some cases, determining the presence or absence of the immunofluorescent CTC markers includes comparing the distinct immunofluorescent staining of CTC candidates with the distinct immunofluorescent staining of a sample cell. In some embodiments, determining the presence or absence of the immunofluorescent CTC markers includes comparing the distinct immunofluorescent staining of CTC candidates with the distinct immunofluorescent staining of WBCs.

In some cases, determining the presence or absence of the immunofluorescent CEC markers includes comparing the distinct immunofluorescent staining of CEC candidates with the distinct immunofluorescent staining of a sample cell. In some embodiments, determining the presence or absence of the immunofluorescent CEC markers includes comparing the distinct immunofluorescent staining of CTC candidates with the distinct immunofluorescent staining of WBCs.

In some embodiments, the morphological characteristics include nucleus size, nucleus shape, cell size, cell shape, and nuclear-to-cytoplasmic ratio. In some embodiments, assessing the morphology of CTC candidates includes assessing the CTC candidates by nuclear detail, nuclear contour, presence or absence of nucleoli, quality of cytoplasm, quantity of cytoplasm, or immunofluorescent staining patterns. In some embodiments, the method further comprises assessing the aggregation characteristics of CTCs, CTC candidates, CTC mimics or CECs.

A person of ordinary skill in the art understands that the morphological characteristics of this disclosure can include any feature, property, characteristic or aspect of a cell that can be determined and correlated with the detection of CTCs, CTC candidates, CTC mimics, or CECs.

The methods of this disclosure can be performed with any microscopic method known in the art. In some embodiments, the method is performed by fluorescent scanning microscopy. In some embodiments the microscopic method provides high-resolution images of CTCs, CTC candidates, CTC mimics or CECs and their surrounding WBCs (*see, e.g.,* Marrinucci D. et al., 2012, Phys. Biol. 9 016003; Nieva J.. et al., 2012, Phys. Biol. 9 016004). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded. The scanned images are analyzed to determine the presence or absence of immunofluorescent markers and to assessment the morphology of the CTC candidates. In some embodiments, microscopic data collection and analysis is conducted in an automated manner.

In some embodiments, the presence or absence of immunofluorescent CTC markers is determined prior to the determination of the presence or absence of immunofluorescent CEC markers. In some embodiments, the presence or absence of immunofluorescent CTC markers and immunofluorescent CEC markers is determined at about the same time. In some embodiments, the presence or absence of immunofluorescent CTC markers is determined in all nucleated cells in a microscopic field of view and the presence or absence of immunofluorescent CEC markers is determined only in CTC candidates.

In some embodiments, the presence or absence of immunofluorescent CTC markers and immunofluorescent sample cell markers is determined at about the same time. In some embodiments the presence or absence of immunofluorescent CTC markers, immunofluorescent CEC markers, and immunofluorescent sample cell markers is determined at about the same time. In some embodiments, the presence or absence of immunofluorescent CTC markers and immunofluorescent sample cell markers is determined prior to the determination of the presence or absence of immunofluorescent CEC markers. In some embodiments, the presence or absence of immunofluorescent CTC markers and immunofluorescent sample cell markers is determined in all cells and the presence or absence of immunofluorescent CEC markers is determined only in CTC candidates. In some embodiments, the presence or absence of immunofluorescent CTC markers, immunofluorescent CEC markers and immunofluorescent sample cell markers is determined in all nucleated cells in a microscopic field of view.

In some embodiments, the determination of the presence or absence of immunofluorescent CTC, CEC, and sample cell markers is determined prior to the assessment of the morphology of CTC candidates. In some embodiments, at least the presence or absence of immunofluorescent CTC markers is determined prior to assessing the morphology of CTC candidates. In some embodiments, the determination of the presence or absence of immunofluorescent CTC, CEC, and sample cell markers occurs at about the same time as the assessment of the morphology of CTC candidates.

In some embodiments, the microscopic field contains CTCs, CTC candidates, CTC mimics and WBCs. In some embodiments, the microscopic field shows at least 1, 5, 10, 20, 50, or 100 CTC candidates. In some embodiments, the microscopic field shows at least 10, 25, 50, 100, 250, 500, or 1,000 fold more WBCs than CTC candidates. In certain embodiments, the microscopic field shows CTC candidates, wherein each CTC candidate is surrounded by at least 10, 50, 100, 150, 200, 250, 500, 1,000 or more WBCs.

In certain embodiments, the microscopy provides a field of view comprising a subpopulation of more than 2, 5, 10, 20, 30, 40 or 50 CTC candidates among the population of nucleated cells, wherein each CTC candidate is surrounded by more than 10, 50, 100, 150 or 200 WBCs. In some embodiments, the microscopy provides a field of view comprising a subpopulation of more than 10 CTC candidates, wherein each CTC candidate is surrounded by more than 200 WBCs.

In some embodiments, a biomarker is considered "present" in a cell if it is detectable above the background signal and noise of the respective detection method used (*e.g.,* 2-fold, 3-fold, 5-fold, or 10-fold higher than the background; 2σ or 3σ over background). In some embodiments, a biomarker is considered "absent" if it is not detectable above the background noise of the detection method used (*e.g.,* <1.5-fold or <2.0-fold higher than the background signal; <1.5σ or <2.0σ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, immunofluorescent CTC or CEC markers, are visible on the WBCs as background signals with fixed heights, even though the respective immunofluorescent CTC or CEC markers are not present in WBCs. Moreover, WBC-specific immunofluorescent markers are visible on CTCs, CTC candidates, CTC mimics and CECs as background signals with fixed heights, even though the markers are not present in CTCs, CTC candidates, CTC mimics or CECs.

A cell is considered positive for an immunofluorescent marker (*i.e.,* the marker is considered present) if its fluorescent signal for the respective marker is significantly higher than the fixed background signal (*e.g.,* 2-fold, 3-fold, 5-fold, or 10-fold higher than the background; 2σ or 3σ over background). For example, a nucleated cell is considered CD 45-positive (CD 45⁺) if its fluorescent signal for CD 45 is significantly higher than the background signal. A cell is considered negative for an immunofluorescent marker (*i.e.,* the marker is considered absent) if the cell's fluorescence signal for the respective marker is not significantly higher than the background signal or noise (*e.g.,* <1.5-fold or <2.0-fold higher than the background signal; *e.g.,* <1.5σ or <2.0σ over background).

The relative expression levels of an immunofluorescent CTC or CEC marker can be expressed by comparing the fluorescence signal of a cell that is positive for the respective marker (*i.e.,* a CTC, CTC candidate, CTC mimic or CEC) with the corresponding fluorescence signal of surrounding cells that are negative for the immunofluorescent CTC or CEC marker (*e.g.,* a WBC). For example, the relative expression of the CTC marker cytokeratin on a given CTC candidate is >5 if the fluorescence signal for cytokeratin on the cell is >5-fold higher than, *e.g.,* the average or median fluorescence signal of surrounding WBCs.

A cell is considered a nucleated cell if it shows a fluorescence signal for a nuclear stain (*e.g.,* DAPI) that is significantly higher than the background signal or noise, *e.g.,* as detected for a non-nucleated platelet cell or for representative cell-free areas on a microscope slide.

In some cases, determining the presence of an immunofluorescent CTC marker in nucleated cells includes identifying nucleated cells having a relative expression of the CTC marker of >2, >3, >4, >5, >6, >7, >8, >9 or >10. In some embodiments, determining the presence of CK in nucleated cells includes identifying nucleated cells having a relative CK expression of >3. *See, e.g.,* **Examples,** **FIGs. 2** and **3****.**

In some cases, determining the presence of an immunofluorescent CEC marker in CTC candidates includes identifying CTC candidates having a relative expression of the CEC marker of >2, >3, >4, >5, >6, >7, >8, >9 or >10. In some embodiments, determining the presence of vWF in CTC candidate includes identifying CTC candidate cells having a relative vWF expression of >6. *See, e.g.,* **Examples,** **FIGs. 2** and **3****.**

The morphological assessment of a nucleated cell, such as the determination of its size or shape, is based on the fluorescence signals of an immunofluorescent marker (*see, e.g.,* Marrinucci D. et al., 2012, Phys. Biol. 9 016003; Nieva J.. et al., 2012, Phys. Biol. 9 016004).

In some embodiments, the CTCs, CTC mimics, CTC candidates and CECs are morphologically distinct from the surrounding WBCs. In some embodiments, assessing the morphology of the CTC candidate comprises comparing the morphological characteristics of the CTC candidate with the morphological characteristics of surrounding WBCs.

In some embodiments, the CTCs, CTC mimics, CTC candidates and CECs are morphologically distinct from each other. *See, e.g.,* **Examples,** **FIG. 4** (CTCs (CK⁺/vWF⁻): top row; CTC mimics (CK⁺/vWF⁺): center row; CEC (CK⁻/vWF+): bottom row). In some embodiments, assessing the morphology of the CTC candidate comprises comparing the morphological characteristics of the CTC candidate with the morphological characteristics of a CTC. In some embodiments, assessing the morphology of the CTC candidate comprises comparing the morphological characteristics of the CTC candidate with the morphological characteristics of a CEC.

Morphological features shared between CTCs and CTC mimics include, for example and without limitation, the presence of distinct and intact nuclei, the presence of nuclei with irregular shapes, the presence of condensed chromatin, a nuclear area that is larger than the nuclear area of WBCs, a cytoplasmic area that is larger than the cytoplasmic area of WBCs, a higher cytoplasmic-to-nuclear ratio relative to WBCs, the presence of aggregates of two or more cytokeratin positive (CK⁺) cells, or combinations thereof.

Morphological features shared between CECs and CTC mimics include, for example and without limitation, the presence of nuclei with irregular shapes, the presence of elongated nuclei, the presence of an elongated cytoplasm, a nuclear area that is larger than the nuclear area of WBCs, a cytoplasmic area that is larger than the cytoplasmic area of WBCs, a higher cytoplasmic-to-nuclear ratio relative to WBCs, the presence of aggregates of two or more Von Willebrand Factor positive (vWF⁺) cells, or combination thereof.

In some embodiments, the (average or mean) nuclear area of CTCs, CTC mimics, or CECs in a microscopic field of view is at least 5%, 10%, 15%, 20%, 25%, 30%, 35%, 40%, 45% or 50% greater than the nuclear area of WBCs.

In some embodiments, the (average or mean) cytoplasmic area of CTCs, CTC mimics or CECs in a microscopic field of view is at least 5%, 10%, 15%, 20%, 25%, 30%, 35%, 40%, 45% or 50% greater than the cytoplasmic area of WBCs.

In some embodiments, the (average or mean) cytoplasmic-to-nuclear ratio of CTCs, CTC mimics or CECs in a microscopic field of view is at least 5%, 10%, 15%, 20%, 25%, 30%, 35%, 40%, 45% or 50% greater than the cytoplasmic-to-nuclear ratio of WBCs.

In some embodiments (also referred to as "high-definition (HD)"-CTC Mimics Assay), the comparison of CTC candidates (*i.e.,* target cells of interest) with surrounding WBCs (*i.e.,* negative control cells) improves the performance of the method, *e.g.,* by increasing the accuracy, specificity, or sensitivity of the method, relative to a method wherein no such comparison is performed. In some embodiments, CTC candidates are compared with surrounding WBCs when determining the presence or absence of a fluorescent marker. In some embodiments, CTC candidates are compared with surrounding WBCs when assessing the morphology of nucleated cells. In some embodiments, CTC candidates are compared with surrounding WBCs when determining the presence or absence of a fluorescent marker and when assessing the morphology of nucleated cells.

The CTC mimics of this disclosure are detected among the nucleated cells of a non-enriched sample based on a combination of distinct immunofluorescent staining and morphological characteristics. In the method of the invention, CTC mimics are identified as CK⁺/vWF⁺/CD45⁻ cells in the non-enriched sample. *See, e.g.,* **Examples,** **FIGs 2-4****.**

In some embodiments, the methods of this disclosure are applied to quantify CEC mimics and to improve the accuracy and sensitivity of CTC quantification.

In some embodiments, the methods of this disclosure further include quantifying the number of CTC candidates in the blood sample.

In some embodiments, the methods further include quantifying the number of CTC mimics in the blood sample.

In some embodiments, the methods of this disclosure further include quantifying the number of CTCs in the blood sample, including i) quantifying the number of CTC candidates in the blood sample, ii) quantifying the number of CTC mimics in the blood sample, and iii) subtracting the number of CTC mimics from the number of CTC candidates to quantify the number of CTCs in the blood sample.

In another aspect, disclosed herein is a method for improving the accuracy and specificity of CTC quantifications in a blood sample, comprising i) quantifying the number of CTC candidates in the blood sample to obtain an approximate CTC count, ii) distinguishing circulating tumor cells (CTCs) from CTC mimics according to a method of this disclosure, iii) quantifying the number of CTC mimics in the blood sample, and iv) subtracting the number of CTC mimics from the number of CTC candidates to improve the accuracy and specificity of CTC quantifications in the blood sample.

In some cases, the methods are used to calculate the concentration of CTCs, CTC candidates, CTC mimics, or CECs in a sample (*e.g.,* in [CEC/ml]). For example, CTC mimics are detected in a human blood sample according to the methods of this disclosure. Next, the ratio of CTC mimics to total nucleated cells (*i.e.,* CTCs, CTC candidates, CTC mimics, CECs plus sample cells, such as WBCs) is determined for a field of vision. Then, the CTC mimic/total nuclear cell ratio is multiplied by the concentration of total nucleated cells in a blood sample (*e.g.,* as determined using a standard automated cell counter) to calculate the concentration of CTC mimics in the blood sample.

In some cases, the methods of this disclosure comprise characterizing the aggregation status of CTC mimics, CTCs, CTC candidates and CECs.

In some cases, the methods of this disclosure comprise the characterization of CTC mimics, CTCs, CTC candidates and CECs with respect to any property, characteristic or aspect observable to a person of ordinary skill in the art. Such characteristics may include, morphological characteristics and cellular dynamics (*e.g.,* cell motility, adhesion to extracellular matrix substrates), immunofluorescence characteristics (*e.g*., intracellular localization of organelles, biomolecules; formation and localization of biomolecular assemblies, such as lipid rafts), metabolic characteristics (*e.g.,* energy metabolism, cellular signaling), genomic characteristics (*e.g.,* gene expression, mRNA splicing), proteomic characteristics (protein expression, localization, post-translational modification). A wide range of methods is available to those skilled in the art to perform a comprehensive characterization of CTC mimics, CTCs, CTC candidates and CECs detected using the methods of this disclosure, including, without limitation, mass spectrometry, gene-chips, FISH, immunocytochemistry, fluorescence microscopy, and the like. In some cases, the methods of this disclosure allow for the further processing and experimentation on samples after the methods of this disclosure have been completed.

In some aspects, the methods of this disclosure are used to detect, quantify and characterize CTCs and CTC mimics in blood samples from human patients suffering from cancer.

This disclosure further describes methods for monitoring an anti-cancer treatment response in a patient, including i) collecting two or more blood samples from the patient at different time-points throughout a treatment period; and ii) quantifying the number of CTC mimics in each blood sample according to a method this disclosure, wherein an increasing number of CTC mimics in the blood samples collected at different time-points throughout the treatment period indicates a positive treatment response in the patient.

This disclosure further describes methods for determining the efficacy of an anti-cancer treatment in a patient, including i) collecting blood samples from a population of patients receiving the anti-cancer treatment, ii) collecting blood samples form a population of patients not receiving the anti-cancer treatment, and iii) quantifying the number of CTC mimics in each blood sample according to a method this disclosure, wherein elevated numbers of CTC mimics in the blood samples from the population of patients receiving the anti-cancer treatment relative to the blood samples from the populations of patients not receiving the anti-cancer treatment indicate that the anti-cancer treatment is efficacious.

In some cases, the methods of this disclosure are used to screen for drugs or to test the efficacy of drug candidates aimed at the treatment of cancer.

The following examples are provided by way of illustration, not limitation.

### EXAMPLES

### Identification, Quantification and Characterization of CTC-like CECs (CTC Mimics) in Non-Enriched Blood Samples from Human Cancer Patients.

This experiment demonstrates that CECs having CTC-like morphology and CK-expression (*i.e.* CTC mimics) are present in the blood of human cancer patients. These CEC mimics can be identified, quantified and further characterized in non-enriched blood samples using a high-definition immunofluorescence assay platform (HD-CTC mimics assay).

The HD-CTC mimics assay uses a combination of distinct immunofluorescent staining and morphological characteristics to identify, quantify and further characterize CEC mimics. The assay is conducted on non-enriched blood samples, as described in the exemplary experiment below.

### Identification of CTC Candidates

First, blood samples were obtained from three confirmed non-small cell lung cancer (NSCLC) patients. CTC candidates were identified in each sample as described, *e.g*., by Marrinucci et al. (2012) Phys Biol 9(1) 016003 or Nieva et al. (2012) Phys Biol 9(1) 016004.

Briefly, blood samples underwent red blood cell lysis followed by monolayer preparation of all nucleated cells on custom glass substrates. After paraformaldehyde (PFA) fixation and methanol permeabilization, cells were incubated with pan anti-cytokeratin antibodies recognizing cytokeratins 1, 4, 5, 6, 7, 8, 10, 13, 18 and 19 and a preconjugated anti-CD45 antibody followed by incubation with an Alexa™ 555-conjugated secondary antibody and DAPI as a nuclear stain. All nucleated cells in the specimen were imaged in multiple fluorescent channels to produce high quality and high resolution digital images that retain fine cytologic detail of nuclear contour and cytoplasmic distribution. Cells that were both cytokeratin positive (CK⁺) and CD45 negative (CD45⁻) were identified using custom computer algorithms and then subjected to morphological analysis (*e.g.,* analysis of their nuclear-to-cytoplasmic ratio). Cells were evaluated by direct review of captured microscopic images and classified as a CTC candidate based on cell morphology (*e.g*., their low nuclear-to-cytoplasmic ratios) and immunophenotype (*e.g*., CK⁺/CD45⁻).

This enrichment-free assay strategy results in high assay sensitivity and specificity, while adding high-definition cytomorphology to enable detailed morphologic characterization of a heterogeneous CTC population. A key advantage of this approach is that multiple analysis parameters can be pursued to identify and characterize specific subpopulations of interest, such as CTC mimics.

### Identification of CEC-Mimics

CEC mimics were identified among the CTC candidates based on a combination of distinct immunofluorescent staining and morphological characteristics.

To identify CEC mimics CTC candidates were probed for expression of the endothelial cell marker Von Willebrand factor (vWF) using a vWF-specific antibody.

An initial assessment of anti-vWF antibody performance was conducted by using a rabbit monoclonal antibody (Sigma Aldrich HPA001815) to detect vWF expression in human umbilical vein endothelial cells (HUVECs, positive control) and H2228 adenocarcinoma cells (negative control). **FIG. 1** shows the results of an anti-vWF antibody-titration experiment. HPA001815 demonstrated optimal performance at concentrations between 0.5 µg/ml and 2.0 µg/ml, where the anti-vWF antibody was found to specifically detect endothelial cells (HUVECs) but not adenocarcinoma cells (H2228).

Blood samples from three confirmed NSCLC patients were processed and analyzed as described above to identify CTC candidate cell populations. Cytokeratin positive (CK⁺) cells were detected and enumerated. Additionally, cells in the non-enriched blood sample were probed for vWF expression using an anti-vWF antibody (HPA001815). Expression of vWF was quantified in all CK positive cells. **FIG. 2** shows the results for three exemplary NSCLC patient samples stained for CK and vWF. All CTC candidates showing relative CK expression >3 are plotted. Negative control H441 cells, which do not express vWF, showed background fluorescence signals of up to 6. Thus, CK expressing cells showing a relative vWF expression >6 (**FIG**. **2****,** dotted line) were considered to be vWF positive. **FIG. 2** demonstrates that subpopulations of cells expressing both CK and vWF were identified in blood samples of all three NSCLC patients.

**FIG. 3** shows a scatter plot of CK vs. vWF expression levels in CK expressing cells (CK rel. expression >3) in a NSCLC patient-derived blood sample. Generally, three subpopulations of cells were distinguishable in patient-derived blood samples based on their CK and vWF expression profiles: CK⁺/vWF⁻ cells, CK⁺/vWF⁺ cells ("double positives") and CK⁻/vWF⁺ cells.

**FIG. 4** shows representative images of CK⁺/vWF⁻ cells (top row), CK⁺/vWF⁺ cells (center row) and CK⁻/vWF⁺ cells (bottom row) and demonstrates that these three cell populations are morphologically distinct. CK⁺/vWF⁻ cells are considered true CTCs, CK⁺/vWF⁺ cells are considered CTC-mimics, *i.e.,* CECs with CTC-like immunofluorescence staining and morphological characteristics, and CK⁻/vWF⁺ cells are considered true CECs.

In summary, this example demonstrates that CTC-mimicking CECs (*i.e.,* CTC-mimics) were identified in the blood sample of human cancer patients using a high-definition immunofluorescence assay platform (HD-CTC mimics assay). CTC mimics were found to be distinguishable from CECs and CTCs based on a combination of distinct immunofluorescent staining and morphological characteristics.

The identification of CTC mimics and their separation from both CTCs and CECs allows for a more specific and accurate determination of CTC and CEC counts in the blood of cancer patients, which enhances the diagnostic and prognostic value of CTC and CEC quantifications. Moreover, CTC-mimics are promising biomarker candidates in their own right. Methods enabling the accurate identification and quantification of CTC mimics can be applied as diagnostic and prognostic tests, *e.g*., to monitor anti-cancer treatment responses in human patients.

## Claims

1. A method of distinguishing circulating tumor cells (CTCs) from CTC mimics based on a combination of distinct immunofluorescent staining and morphological characteristics, wherein the method comprises:
(a) determining the presence or absence of one or more immunofluorescent CTC markers in nucleated cells in a non-enriched blood sample to detect a CTC candidate, wherein the non-enriched blood sample is a sample not enriched for any specific population or subpopulation of nucleated cells;
(b) determining the presence or absence of one or more immunofluorescent circulating endothelial cell (CEC) markers in the CTC candidate;
(c) determining the presence or absence of one or more immunofluorescent sample cell markers in the CTC candidate; and
(d) assessing the morphology of the CTC candidate,
wherein the immunofluorescent CTC markers comprise a cytokeratin (CK),
wherein the immunofluorescent CEC markers comprise Von Willebrand factor (vWF),
wherein the immunofluorescent sample cell markers comprise cluster of differentiation 45 (CD45),
wherein the distinct immunofluorescent staining of CTC mimics includes the presence of the immunofluorescent CTC marker CK (CK+), the presence of the immunofluorescent CEC marker vWF (vWF+), and the absence of the immunofluorescent sample cell marker CD45 (CD45-), and
wherein the distinct immunofluorescent staining of CTCs includes the presence of CK (CK+), the absence of vWF (vWF-), and the absence of CD45 (CD45-).

2. The method of claim 1, wherein the CK includes cytokeratin 1, 4, 5, 6, 7, 8, 10, 13, 18 or 19, or a combination thereof.

3. The method of claim 1 or 2, wherein determining the presence of CK in the CTC candidate comprises identifying nucleated cells having a relative CK expression of >3, wherein the relative expression level is expressed by comparing the fluorescence signal of a cell that is positive for CK with the corresponding fluorescence signal of surrounding cells that are negative for CK.

4. The method of claim 1, wherein the immunofluorescent CEC markers include CD31, CD34, CD105, or CD146.

5. The method of claim 1, wherein determining the presence of vWF in the CTC candidate comprises identifying CTC candidates having a relative vWF expression of >6, wherein the relative expression level is expressed by comparing the fluorescence signal of a cell that is positive for vWF with the corresponding fluorescence signal of surrounding cells that are negative for vWF.

6. The method of claim 1, wherein assessing the morphology of the CTC candidate comprises assessing the CTC candidate by nuclear detail, nuclear contour, presence or absence of nucleoli, quality of cytoplasm, quantity of cytoplasm, or immunofluorescent staining patterns.

7. The method of claim 1, wherein the blood sample was obtained from a non-small cell lung cancer (NSCLC) patient.

8. The method of claim 1, wherein the method is performed by fluorescent scanning microscopy.

9. The method of claim 8, wherein the microscopy provides a field of view comprising more than 2, 5, 10, 20, 30, 40 or 50 CTC candidates, wherein each CTC candidate is surrounded by more than 10, 50, 100, 150 or 200 white blood cells (WBC).

## Patentansprüche

1. Verfahren zur Unterscheidung zirkulierender Tumorzellen (CTC) von CTC-Mimetika, basierend auf einer Kombination von ausgeprägten Immunfluoreszenzfärbungen und morphologischen Merkmalen, wobei das Verfahren umfasst:
(a) Bestimmen des Vorliegens oder Fehlens eines oder mehrerer immunfluoreszierender CTC-Marker in kernhaltigen Zellen in einer nicht angereicherten Blutprobe zum Nachweis eines CTC-Kandidaten, wobei die nicht angereicherte Blutprobe eine Probe ist, die nicht für eine spezifische Population oder Subpopulation kernhaltiger Zellen angereichert ist;
(b) Bestimmen des Vorliegens oder Fehlens eines oder mehrerer immunfluoreszierender zirkulierender Endothelzell (CEC)-Marker in dem CTC-Kandidaten;
(c) Bestimmen des Vorliegens oder Fehlens eines oder mehrerer immunfluoreszierender Probenzell-Marker in dem CTC-Kandidaten; und
(d) Beurteilen der Morphologie des CTC-Kandidaten,
wobei die immunfluoreszierenden CTC-Marker ein Cytokeratin (CK) umfassen,
wobei die immunfluoreszierenden CEC-Marker den von-Willebrand-Faktor (vWF) umfassen,
wobei die immunfluoreszierenden Probenzell-Marker das Cluster der Differenzierung 45 (CD45) umfassen,
wobei die ausgeprägte Immunfluoreszenzfärbung der CTC-Mimetika das Vorliegen des immunfluoreszierenden CTC-Markers CK (CK+), das Vorliegen des immunfluoreszierenden CEC-Markers vWF (vWF+) und das Fehlen des immunfluoreszierenden Probenzellmarkers CD45 (CD45-) beinhaltet, und
wobei die ausgeprägte Immunfluoreszenzfärbung der CTCs das Vorliegen von CK (CK+), das Fehlen von vWF (vWF-) und das Fehlen von CD45 (CD45-) beinhaltet.

2. Verfahren nach Anspruch 1, wobei das CK Cytokeratin 1, 4, 5, 6, 7, 8, 10, 13, 18 oder 19 oder eine Kombination davon umfasst.

3. Verfahren nach Anspruch 1 oder 2, wobei das Bestimmen des Vorliegens von CK in dem CTC-Kandidaten die Identifizierung von kernhaltigen Zellen mit einer relativen CK-Expression von >3 umfasst, wobei das relative Expressionsniveau durch Vergleich des Fluoreszenzsignals einer Zelle, die positiv ist für CK, mit dem entsprechenden Fluoreszenzsignal von umgebenden Zellen, die für CK negativ sind, exprimiert wird.

4. Verfahren nach Anspruch 1, wobei die immunfluoreszierenden CEC-Marker CD31, CD34, CD105 oder CD146 beinhalten.

5. Verfahren nach Anspruch 1, wobei das Bestimmen des Vorliegens von vWF in dem CTC-Kandidaten die Identifizierung von CTC-Kandidaten mit einer relativen vWF-Expression von >6 umfasst, wobei das relative Expressionsniveau durch Vergleich des Fluoreszenzsignals einer Zelle, die positiv ist für vWF, mit dem entsprechenden Fluoreszenzsignal von umgebenden Zellen, die für vWF negativ sind, exprimiert wird.

6. Verfahren nach Anspruch 1, wobei das Beurteilen der Morphologie des CTC-Kandidaten ein Beurteilen des CTC-Kandidaten nach Kerndetail, Kernkontur, Vorliegen oder Fehlen von Nukleoli, Qualität des Zytoplasmas, Quantität des Zytoplasmas oder immunfluoreszierenden Färbemustern umfasst.

7. Verfahren nach Anspruch 1, wobei die Blutprobe von einem Patienten mit nichtkleinzelligem Lungenkrebs (NSCLC) erhalten wurde.

8. Verfahren nach Anspruch 1, wobei das Verfahren mittels Fluoreszenz-Rastermikroskopie durchgeführt wird.

9. Verfahren nach Anspruch 8, wobei die Mikroskopie ein Sichtfeld liefert, das mehr als 2, 5, 10, 20, 30, 40 oder 50 CTC-Kandidaten umfasst, wobei jeder CTC-Kandidat von mehr als 10, 50, 100, 150 oder 200 weißen Blutzellen (WBZ) umgeben ist.

## Revendications

1. Méthode pour distinguer les cellules tumorales circulantes (CTCs) des mimétiques de CTC basée sur une combinaison de coloration immunofluorescente distincte et de caractéristiques morphologiques, dans laquelle la méthode comprend
a) déterminer la présence ou l'absence d'un ou plusieurs marqueurs CTC immunofluorescents dans les cellules nucléées dans un échantillon de sang non enrichi pour détecter un candidat CTC, l'échantillon de sang non enrichi étant un échantillon non enrichi pour une population ou une sous-population spécifique de cellules nucléées ;
b) déterminer la présence ou l'absence d'un ou plusieurs marqueurs immunofluorescents des cellules endothéliales circulantes (CEC) dans le candidat CTC ;
c) déterminer la présence ou l'absence d'un ou plusieurs marqueurs cellulaires immunofluorescents de l'échantillon dans le candidat CTC ; et
d) évaluer la morphologie du candidat CTC,
dans laquelle les marqueurs CTC immunofluorescents comprennent une cytokératine (CK),
dans laquelle les marqueurs immunofluorescents de la CEC comprennent le facteur de von Willebrand (vWF),
dans laquelle les marqueurs cellulaires immunofluorescents de l'échantillon comprennent le groupe de différenciation 45 (CD45),
dans laquelle la coloration immunofluorescente distincte des mimétiques CTC comprend la présence du marqueur CTC immunofluorescent CK (CK+), la présence du marqueur CEC immunofluorescent vWF (vWF+) et l'absence du marqueur cellulaire immunofluorescent CD45 (CD45-) de l'échantillon, et
dans laquelle la coloration immunofluorescente distincte des CTC comprend la présence de CK (CK+), l'absence de vWF (vWF-) et l'absence de CD45 (CD45-).

2. Méthode selon la revendication 1, dans laquelle la CK comprend les cytokératines 1, 4, 5, 6, 7, 8, 10, 13, 18 ou 19, ou une combinaison de celles-ci.

3. Méthode selon la revendication 1 ou 2, dans laquelle la détermination de la présence de CK dans le candidat CTC comprend l'identification de cellules nucléées ayant une expression relative de CK >3, dans laquelle le niveau d'expression relative est exprimé en comparant le signal de fluorescence d'une cellule qui est positive pour la CK avec le signal de fluorescence correspondant des cellules environnantes qui sont négatives pour la CK.

4. Méthode selon la revendication 1, dans laquelle les marqueurs CEC immunofluorescents comprennent CD31, CD34, CD105 ou CD146.

5. Méthode selon la revendication 1, dans laquelle la détermination de la présence de vWF dans le candidat CTC comprend l'identification des candidats CTC ayant une expression relative de vWF >6, dans laquelle le niveau d'expression relative est exprimé en comparant le signal de fluorescence d'une cellule qui est positive pour le vWF avec le signal de fluorescence correspondant des cellules environnantes qui sont négatives pour le vWF.

6. Méthode selon la revendication 1, dans laquelle l'évaluation de la morphologie du candidat CTC comprend l'évaluation du candidat CTC par détail nucléaire, contour nucléaire, présence ou absence de nucléole, qualité du cytoplasme, quantité de cytoplasme ou modèles de coloration immunofluorescente.

7. Méthode selon la revendication 1, selon laquelle l'échantillon de sang a été obtenu d'un patient atteint d'un cancer du poumon non à petites cellules (CPNPC).

8. Méthode selon la revendication 1, dans laquelle la méthode est réalisée par microscopie à balayage fluorescent.

9. Méthode selon la revendication 8, dans laquelle la microscopie fournit un champ de vision comprenant plus de 2, 5, 10, 20, 30, 40 ou 50 candidats CTC, dans laquelle chaque candidat CTC est entouré par plus de 10, 50, 100, 150 ou 200 globules blancs (GBs).
